# EUROPEAN PATENT SPECIFICATION

(11) **EP 0 824 213 B1**
(45) Date of publication and mention of the grant of the patent: **27.08.2003**
(21) Application number: 97102952.5
(22) Date of filing: 22.02.1997
(51) Int. Cl.: G01N 33/48, A61K 35/18

(54) **Method for fractionating red blood cells and antibacterial materials or bacterial proliferation inhibitors produced thereby**
Verfahren zur Fraktionierung von roten Blutzellen, und so hergestellte antibakterielle Wirkstoffe oder bakterielle Fortpflanzungshemmstoffe
Méthode pour le fractionnement des globules rouges et matériaux antibactériens ou inhibiteurs de prolifération bactérienne ainsi produit

(30) Priority: 15.08.1996 JP 21555296
(43) Date of publication of application: 18.02.1998
(73) Proprietor: Matsumoto, Tsukasa, Tokyo 143 (JP)
(72) Inventor: Matsumoto, Tsukasa, Tokyo 143 (JP)
(74) Representative: Müller, Enno, Dipl.-Ing.

(56) References cited:
- WO-A-93/10153
- US-A- 3 858 795
- US-A- 4 224 942
- US-A- 4 255 256
- US-A- 4 765 899
- US-A- 5 397 479
- W. LEE HAND: "Inhibition of cell-free oxidative bactericidal activity by erythrocytes and hemoglobin" INFECTION AND IMMUNITY, vol. 44, no. 2, May 1984, pages 465-468, XP002062176
- J. M. GUTTERIDGE: "Superoxide dismutase (erythrocuprein) and free radicals in clinical chemistry" ANNALS OF CLINICAL BIOCHEMISTRY, vol. 13, no. 3, May 1976, pages 393-398, XP002062177
- F. R. VENEZIO, C. DIVINCENZO, R. SHERMAN, M. REICHMAN, T. C. ORIGITANO, K. THOMPSON, O. H. REICHMAN: "Bactericidal effects of photoradiation therapy with hematoporphyrin derivative" JOURNAL OF INFECTIOUS DISEASES, vol. 151, no. 1, January 1985, pages 166-169, XP002062178
- P. MARTINETTO, M. GARIGLIO, G. F. LOMBARD, B. FISCELLA, F. BOGGIO: "Bactericidal effects induced by laser irradiation and hematoporphyrin against Gram-positive and Gram-negative microorganisms" DRUGS UNDER EXPERIMENTAL AND CLINICAL RESEARCH, vol. 12, no. 4, 1986, pages 335-342, XP002062179
- Z. MALIK, H. LADAN, Y. NITZAN, B. ENRENBERG: "The bactericidal activity of a deuteroporphyrin-hemin mixture on gram-positive bacteria: A microbiological and spectroscopic study" JOURNAL OF PHOTOCHEMISTRY AND PHOTOBIOLOGY B: BIOLOGY, vol. 6, no. 4, 1990, pages 419-430, XP002062180

## Description

### BACKGROUND OF THE INVENTION

### 1.Field of the Invention

The present invention relates to methods for fractionating red blood cells (RBC) of human blood into several fractions having different functions. Further the present invention relates to specific materials produced by such method, which are possessed of antibacterial properties or inhibitory against bacterial proliferation.

### 2.Prior Art

Although it has been conventionally known that red blood cells of human blood act as carriers for carrying a large amount of oxygen and carbon dioxide at a high speed, the other functions of red blood cells have not yet been known completely.

US-A-4,255,256 comprises the step that a human blood sample is mixed with dextran aqueous solution. Then said mixture is centrifuged so as to fractionate this blood sample into three layers, the upper, intermediate and lower layers. The upper layer is separated and collected from the other layers.

US-A-4,765,899 discloses an apparatus for continuous separation of a leukocyte/platelet enriched fraction from whole blood. The corresponding method is characterised in that human blood is mixed with dextran aqueous solution and said mixture is exposed to a flow sedimentation so as to fractionate the blood into two layers.

US-A-5,397,479 discloses a method for enrichment of white blood cells from whole blood. In the first step of this method human blood is mixed with dextran aqueous solution and hypertonic solution. Then said mixture is maintained stationary so as to fractionate the blood.

US-A-4,224,942 discloses a cell fractionating method, wherein human blood is mixed with dextran aqueous solution and said mixture is exposed to a velocity sedimentation so as to fractionate the mixture into at least two samples. The corresponding red blood cells fractionated are washed with isotonic solution.

### SUMMARY OF THE INVENTION

It is therefore a primary object of this invention to find new functions of red blood cells which are greater parts of human blood.
It is still more specific object of this invention to provide methods for fractionaing red blood cells into different functions, which method can be used for such research.

In order to achieve the above objects, the inventor of the present invention has been performed various researches for fractionating red blood cells of human blood into several fractions. The inventor found that the tested blood could be fractionated into three layers after the blood was added with dextran and then maintained under a certain condition for a certain period. The fractionated blood cells contained these three layers provided different functions on bacteria, respectively. The present invention is based on this knowledge.

The above described objects are accomplished by the method for producing a fraction including antibacterial red blood cells, which comprises following steps; (a)human blood sample is mixed with dextran aqueous solution and maintained stationarily for 60 to 75 min to fractionate this blood sample into three layers; (b)the upper layer is separated from the other layers; and (c)the upper layer is treated with hypotonic solution for a short period and then added with hypertonic solution.

Accordingly, the methods provided by the present invention can easily fractionate three different blood fractions including red blood cells as a main component and having different functions. These three different fractions may be applied to several clinical tests such as antibacterial test and the like.

These and other objects and many of the attendant advantages of this invention will be readily appreciated as the same becomes better understood by reference to the following detailed description when considered in connection with the accompanying drawing.

### BRIEF DESCRIPTION OF THE DRAWING

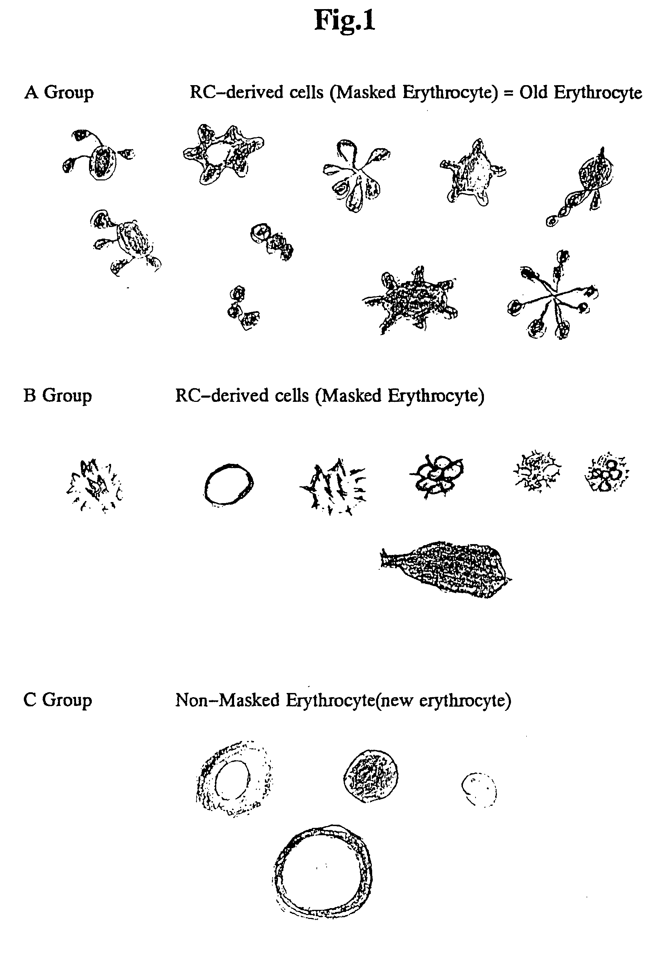

Fig. 1 is a diagrammatic illustration of changes in the shape of red blood cells included in the fractions provided by the methods according to the present invention.

### DETAILED DESCRIPTION OF THE PREFERRED EMBODIMENT

First, according to one preferred embodiment of the present invention, human blood (including all blood ingredients) is mixed with dextran aqueous solution. This dextran aqueous solution used in the present invention is preferably selected from 5 to 10 w/v% of dextran aqueous solution or physiological saline solution, more preferably 5 to 10 w/v% of dextran physiological saline solution, most preferably 5 to 8 w/v% of dextran physiological saline solution. This dextran aqueous solution to be added into the blood sample is preferably one to twice the volume of the blood sample, more preferably 1.5 times. The mixture of the blood and the dextran aqueous solution is sufficiently stirred and then remained stationarily for 60 to 75 minutes. The mixture becomes forming three layers (upper, intermediate, and lower layers).

The upper layer is separated from the other layers by means of a pipette. The separated layer is treated with hypotonic solution for a short period and then added with hypertonic solution. Finally, one fraction containing red blood cells providing antibacterial activity is produced. In practical manner, the upper layer is subjected to centrifugal separation to collect blood cells. The separated blood cell sample is further added with hypotonic solution, and after a short period, added with hypertonic solution to provide isotonic solution. In the present invention, a saponin solution is preferably used for the hypotonic solution, but any materials capable of making the hypotonic solution to isotonic may be used for the hypertonic solution. Such short period for remaining the blood cell sample in the hypotonic solution is preferably about 30 seconds. If the treatment period in the hypotonic solution is shorter, the produced sample will not be antibacterial. On the other hand, if longer, hemolysis will excessively progress and thus the sample includes less red blood cells.

The intermediate and lower layers may be selectively taken from the separated layers by means of a pipette, respectively.

Such prepared three red blood cell fractions are usually added into liquid medium such as RPMI-1640, MEM, BME, Ham F12, MCDB104, MCDB153, and so on to be applied to various researches and studies such as observation of act on various bacteria.

One experiment wherein specific bacteria were inoculated upon the above hypotonic treated upper layer indicated that the red blood cells contained in the upper layer attacked upon these bacteria while the leucocytes contained in the upper layer did not act on these bacteria at all. In other words, the red blood cells contained in the upper layer provide antibacterial function. When the same bacteria were inoculated upon the intermediate layer, this experimet resulted in that proliferation of bacteria was remarkably inhibited. Also when the lower layer was subjected to the similar experiment, this test resulted in no action on the inoculated bacteria.

According to another preferred embodiment of the present invention, the same three red blood cell fractions; the upper, intermediate and lower layers, were fractionated and prepared as liquid medium in the same manner as above. Incubated leucocyte sample taken from another human blood was added into the upper and intermediate layers, respectively. In order to observe activation on bacteria in these fractionated samples, specific bacteria were immediately added into the upper and intermediate layers including the incubated leucocytes, respectively. These samples were observed three times at intervals of 24 hours; i.e., after 24, 48, 72 hours. These observations resulted in that proliferation of bacteria was remarkably inhibited. Reference tests using commercial available antibiotics were carried out on the same occasion to compare with the above results according to the present invention. This comparison also proved that the upper and intermediate layer samples provided superior inhibitory on bacteria rather than all antibiotics.

As is clear from these experimental tests, specific materials having stronger inhibitory effect on proliferation of bacteria rather than any existing antibiotics are generated in the liquid medium samples composed of the upper layer including red blood cells and incubated leucocytes added thereto, and the intermediate layer including red blood cells and incubated leucocytes added thereto, and further their activated condition is maintained stably for a long period.

It should be understood that such inhibitory materials are either cell-products composed of the red blood cells contained in each layer and the leucocytes added thereto, or by-products secreted out of the blood cells. It should be understood that such by-products are the first type materials secreted out of the red blood cells in the upper or intermediate layer acted by the incubated leucocytes added thereto; the second type materials generated when the incubated leucocytes are broken or secreted out of the incubated leucocytes; or the third type materials generated by the cooperation between the red blood cells of each fraction and the incubated leucocytes. In order to specify such inhibitory materials, the cultured solution of the red blood cell fraction added with the incubated leucocytes is subjected to analysis via filtering and extracting processes.

The present invention will be further understood by the following example. However, the present invention is not limited to this example.

### Example

Fresh human blood 10 ml of a healthy person was added with dextran solution 15 ml (which was prepared by dissolving 7.0 g of Dextran 70 manufactured by Tokyo Kasei Co.,Ltd in 100 ml of physiological saline solution), and sufficiently stirred by a pipette, and then maintained stationarily for 60 to 75 min. This sample solution was separated into three layers, the upper, intermediate, and lower layers.

The upper layer was subjected to a centrifugal separation to precipitate blood cells (red blood cells and leucocytes). This precipitated part was added with hypotonic solution (RLB, produced by Harajuku Clinic) 30 ml, and after 30 sec, added with hypertonic solution (BELMAR, produced by Harajuku Clinic) 10 ml. This mixture was subjected to a centrifugal separation to collect blood cells. Thus collected blood cells were further added with liquid medium (RPMI-1640) 3 ml. This solution was referred to "sample A".

Four drops of the intermediate layer were taken up by a pippet. This drop-solution was added with liquid medium (RPMI-1640) 3 ml. This solution was referred to "sample B".

A drop of the lower layer was taken up by a pippet. This drop-solution was added with liquid medium (RPMI-1640) 3 ml. This solution was referred to "sample C".

### Test and Results

A small amount of Pseudomonas aeruginosa was inoculated upon the samples A, B, C and RPMI-1640 (as a control sample), respectively. These samples were remained for 5 to 6 hours at room temperature, and then incubated under the condition of 5% CO₂, 37°C. Thus resulted samples A, B, C and control sample were observed through a microscope to know the action of the samples A, B, C and control sample on the inoculated bacteria.

The micoscopic observation resulted in that the sample A included some leucocytes in addition to red blood cells, and the red blood cells were changed into various shapes as shown in Fig. 1. According to the observation of the sample A incubated for six housrs, the leucocytes did not positively act on the inoculated bacteria at all, while the red blood cells changed in their shapes moved towoard the inoculated bacteria and thus the bacteria were prohibited from moving freely.

The micoscopic observation resulted in that the sample B also included a small amount of leucocytes in addition to red blood cells, and the red blood cells were changed into various shapes as shown in Fig. 1. According to the observation of the sample B incubated for six housrs, the inoculated bacteria were remarkably decreased in comparison with the control sample.

According to the micoscopic observation of the sample C, the sample C included only red blood cells which were changed into various shapes. The counted number of the inoculated bacteria in the sample C were substantialy equal to that of the control sample.

As is clear from the above description, the methods provided by the present invention can easily fractionate three different blood fractions including red blood cells as a main component and having different functions. These three different fractions may be applied to several clinical tests such as antibacterial test and the like.

## Claims

1. Method for producing a fraction including antibacterial red blood cells, which comprises following steps:
(a) mixing human blood sample with dextran aqueous solution and treating said mixture so as to fractionate this blood sample into three layers, the upper, intermediate, and lower layers,
(b) separating and collecting the upper layer from the other layers,
**characterized in that** in step (a) the treating of said mixture is realized by maintaining said mixture stationarily for 60 to 75 minutes and in a step (c) the upper layer sample is treated with hypotonic solution for a short period of about 30 seconds and then hypertonic solution is added into said upper layer sample.

2. Method according to claim 1, **characterized in that** in step (c) an isotonic solution is produced by the adding of the hypertonic solution into the upper layer sample and incubated leucocytes are added into said isotonic solution.

3. Antibacterial material included in the solution produced by following steps:
(a) mixing human blood sample with dextran aqueous solution and treating said mixture so as to fractionate this blood sample into three layers, the upper, intermediate, and lower layers,
(b) separating and collecting the upper layer from the other layers;
**characterized in that** in step (a) the treating of said mixture is realized by maintaining said mixture stationarily for 60 to 75 minutes and in a step (c) the upper layer sample is treated with hypotonic solution for a short period for about 30 seconds and then hypertonic solution is added into said upper layer sample.

4. Antibacterial material according to claim 3, **characterized in that** in step (c) an isotonic solution is produced by the adding of the hypertonic solution into the upper layer sample and incubated leucocytes are added into said isotonic solution.

## Patentansprüche

1. Verfahren zur Herstellung einer Fraktion aufweisend antibakterielle rote Blutzellen, welches die folgenden Schritte aufweist:
(a) Mischen einer menschlichen Blutprobe mit einer wässrigen Dextranlösung und behandeln der Mischung derart, dass das Blut in drei Schichten, eine obere, eine mittlere und eine untere Schicht, fraktioniert wird;
(b) Abteilung und Einsammlung der oberen Schicht von anderen Schichten,
**dadurch gekennzeichnet, dass** im Schritt (a) die Behandlung der Mischung durch Stillstehen-Lassen der Mischung für 60 bis 75 Minuten durchgeführt wird und in einem Schritt (c) die obere Schichtprobe mit hypotonischer Lösung behandelt wird für einen kurzen Zeitraum von etwa 30 Sekunden und dann eine hypertonische Lösung in die obere Schicht hinzugegeben wird.

2. Verfahren nach Anspruch 1, **dadurch gekennzeichnet, dass** in Schritt (c) eine isotonische Lösung hergestellt wird durch Hinzufügen der hypertonischen Lösung in die obere Schicht und dass inkubierte Leukozyten in die isotonische Lösung hinzugegeben werden.

3. Antibakterieller Stoff einbezogen in einer in folgenden Schritten hergestellten Lösung:
(a) Mischen einer menschlichen Blutprobe mit einer wässrigen Dextranlösung und Behandlung der Mischung derart, dass die Blutprobe fraktioniert wird in drei Schichten, eine obere, mittlere und eine untere Schicht;
(b) Abteilung und Einsammlung der oberen Schicht von den anderen Schichten
**dadurch gekennzeichnet, dass** in Schritt (a) die Behandlung der Mischung durchgeführt wird durch Stillstehen-Lassen der Mischung für 60 bis 75 Minuten und in einem Schritt (c) die obere Lagenprobe mit hypotonischer Lösung für einen kurzen Zeitraum von etwa 30 Sekunden behandelt wird und dann die hypertonische Lösung in die obere Schichtprobe hinzugegeben wird.

4. Antibakterieller Stoff nach Anspruch 3, **dadurch gekennzeichnet, dass** in Schritt (c) eine isotonische Lösung hergestellt wird durch Hinzufügen der hypertonischen Lösung in die obere Schichtprobe und dass inkubierte Leukozyten in die isotonische Lösung hinzugegeben werden.

## Revendications

1. Procédé pour produire une fraction comprenant des globules rouges antibactériens, qui comprend les étapes suivantes consistant à :
(a) mélanger un échantillon de sang humain à une solution aqueuse de dextrane et à traiter ledit mélange afin de fractionner cet échantillon de sang en trois couches, les couches supérieure, intermédiaire, et inférieure,
(b) séparer et à recueillir la couche supérieure des autres couches,
**caractérisé en ce que** à l'étape (a) le traitement dudit mélange est réalisé en maintenant ledit mélange de manière stable pendant 60 à 75 minutes et **en ce que** à une étape (c) l'échantillon de la couche supérieure est traité avec une solution hypotonique pendant un court laps de temps d'environ 30 secondes et ensuite une solution hypertonique est ajoutée dans ledit échantillon de la couche supérieure.

2. Procédé selon la revendication 1, **caractérisé en ce que** à l'étape (c) une solution isotonique est produite en ajoutant une solution hypertonique dans l'échantillon de la couche supérieure et des leucocytes incubés sont ajoutés dans ladite solution isotonique.

3. Matériau antibactérien inclus dans la solution produit par les étapes suivantes consistant à :
(a) mélanger un échantillon de sang humain à une solution aqueuse de dextrane et à traiter ledit mélange afin de fractionner cet échantillon de sang en trois couches, les couches supérieure, intermédiaire, et inférieure,
(b) séparer et à recueillir la couche supérieure des autres couches ;
**caractérisé en ce que** à l'étape (a) le traitement dudit mélange est réalisé en maintenant ledit mélange de manière stable pendant 60 à 75 minutes et **en ce que** à une étape (c) l'échantillon de la couche supérieure est traité avec une solution hypotonique pendant un court laps de temps d'environ 30 secondes et ensuite une solution hypertonique est ajoutée dans ledit échantillon de la couche supérieure.

4. Matériau antibactérien selon la revendication 3, **caractérisé en ce que** à l'étape (c) une solution isotonique est produite en ajoutant une solution hypertonique dans l'échantillon de la couche supérieure et des leucocytes incubés sont ajoutés dans ladite solution isotonique.
